# EUROPEAN PATENT SPECIFICATION

(11) **EP 4 470 525 B1**
(45) Date of publication and mention of the grant of the patent: **26.11.2025**
(21) Application number: 24178509.6
(22) Date of filing: 28.05.2024
(51) Int. Cl.: A61K 9/00, A61K 8/02, A61K 8/66, A61K 8/86, A61K 9/06, A61K 9/70, A61K 38/40, A61K 38/47, A61K 47/10, A61K 47/12, A61K 47/34, A61P 17/10, C07K 14/79, C12N 9/36

(54) **NEW STABLE GEL FORMULATIONS FOR THE TREATMENT OF ACNE VULGARIS**
NEUE STABILE GELFORMULIERUNGEN ZUR BEHANDLUNG VON AKNE VULGARIS
NOUVELLES FORMULATIONS DE GEL STABLES POUR LE TRAITEMENT DE L'ACNÉ VULGAIRE

(30) Priority: 30.05.2023 US 202363504798 P; 28.06.2023 IT 202300013428
(43) Date of publication of application: 04.12.2024
(73) Proprietor: Bioseutica B.V., 3899 BD Zeewolde (NL)
(72) Inventor: FERRARI, Valerio Maria, 20129 Milan (IT); GAO, Yuncai, Abbotsford, V2S 8M8 (CA); FORTINA, Giacomo, 28823 GHIFFA (VB) (IT); CALIZZI, Alessandro, 21046 MALNATE (VA) (IT); ASTRUA, Carlo, 28922 VERBANIA (IT); VIGILANTE, Mario, 22100 COMO (IT); LLOYD SANTUCCI, Zefferino, V2S 5X9 Abbotsford (CA); GRISENTI, Paride, 20141 MILANO (IT)
(74) Representative: Bianchetti & Minoja with Trevisan & Cuonzo IPS SRL

(56) References cited:
- WO-A1-01/89540
- WO-A1-2005/048962
- US-B2- 9 757 328

## Description

### Field of invention

The present invention relates to compositions and methods for the preparation of a topical gel for the treatment of *acne vulgaris* and, in particular, to topical gel compositions comprising lysozyme and ovotransferrin as active ingredients and ethoxylated dimeric urethanes as gelling agents.

### Background of the invention

Lysozyme C type is a natural protein with a molecular weight of 14,4 kDa extracted from hen eggs white (HEW) endowed with antimicrobial properties. This compound, active mainly on Gram-positive bacteria and on some viruses, is not active on Gram-negative bacteria. Ovotransferrin, which has a molecular weight of 76 kDa, is another natural protein present in the HEW. This protein belongs to the family of transferrins; it possesses antibacterial, antioxidant and immunomodulatory activities due to its ability to bind iron (Fe³⁺) by sequestering it in bacterial biochemical processes. Ovotransferrin has antibacterial activity against a wide spectrum of bacteria, including *E. coli* (Schade and Caroline, 1944; Ko *et al.,* 2008), and lysozyme has been shown to improve this activity (Ko *et al.,* 2009). Thus, the complex of lysozyme and ovotransferrin is expected to have enhanced antibacterial activity against a wide range of bacteria, including Gram-positive and Gram-negative species (Journal of Microbiological Methods Volume 154, November 2018, Pages 19-24, doi.org/10.1016/j.mimet.2018.10.001).

*Acne vulgaris*, a chronic inflammatory disorder of the pilosebaceous apparatus is triggered by *Cutibacterium acnes* (*C. acnes* formerly called *Propionibacterium acnes*) in adolescence, under the influence of the hormonal steroid dehydroepiandrosterone, the active metabolite of androsterone.

*C. acnes* is a Gram-positive, aerotolerant anaerobic bacillus able to produce propionic acid as a metabolic by-product and it is part of the normal skin microflora. However, results from recent studies clearly indicate that acne infections also involve other relevant changes in the skin microbiota, particularly an increase of the presence of other bacteria such as *Cutibacterium granulosum, Staphylococcus epidermidis, Streptococcus, Malassezia species, Firmicutes* and *Proteobacteria* (J Clin Med. 2019 Jul 7;8(7):987, doi: 10.3390/jcm8070987).

*Proteobacteria* comprise several known human pathogens, including for example the *Escherichia, Salmonella* and *Helicobacter* class; these Gram-negative microorganisms are present in various body sites, such as skin, oral cavity, tongue and vaginal tract, as well as in the human gut and stool.

The topical application of lysozyme preparation to effectively treat purulent skin infections caused by acne has been known since 1963: an aqueous formulation at pH 5.5 containing lysozyme HCl (2%) and excipients such as sucrose monolaurate (2%), sucrose distearate (4%) and polyethylene glycol-20 (20%) was disclosed in Aromi, Saponi, Cosmetici (1963), 45, 4758).

The antimicrobial properties of this formulation containing lysozyme HCl for the treatment of *acne vulgaris* is not fully satisfactory since it is limited against Gram-positive microorganisms; therefore several other formulations have been developed with the aim to extend the antimicrobial activity.

Topical formulations of lysozyme (10-30%) were developed in association with proteases (10-30% bromelin, papain) and a chelating agent such as ethylenediaminetetraacetic acid (ETDA; 2-60%) (HU57608). In this case, EDTA was used to expand the antimicrobial effect against Gram-negative bacteria, amoeba and fungi (Advances in Wound Care, 2015 July 1, 4, 7, 415-421). A similar cosmetic composition, containing lysozyme and other enzymes such as elastase, α-lipase, and/or α-amylase and optionally EDTA as complexing agent, is disclosed in DE4305460: the antimicrobial activity of the formulation was confirmed to be useful for the prevention and treatment of acne caused by *S. aureus, S. epidermidis, E. coli, B. subtilis,* and *Klebsiella. In vitro,* the antimicrobial activity against Gram-negative bacteria (i.e. E. coli and Klebsiella) was enhanced by EDTA. Another gel formulation containing lysozyme useful for protecting skin against infections caused by *S. aureus, P. aeruginosa, S. epidermidis, Propionibacterium* strains and other drug resistant bacteria, is disclosed in CN1709503. Said gel formulation is prepared by mixing human lysozyme with gel matrix containing polyethylene glycol 400 or sodium carboxymethyl cellulose, stearyl alcohol, glycerin, sodium pyrrolidine cuprate, methyl paraben and distilled water. These formulations provide the use of a recombinant human lysozyme and not of a lysozyme C type, proteins which are different in the primary structure and other characteristics, such as antibacterial activity and thermal stability, the recombinant lysozyme being the most stable to heating and having higher antibacterial activity (Front Nutr. 2022; 9: 833618, doi: 10.3389/fnut.2022.833618).

Following the above-mentioned approach to use the combined action of lysozyme C type or of a recombinant human lysozyme with proteases and of a C1-C4 polysaccharide (chitosan or pullulan; Formula 1), WO2008124764 discloses the use of different compositions, including a gel composition for the use in dermatology and cosmetology and optionally one or more additional active agents selected from the family of transferring proteins (such as lactoferrin and ovotransferrin), a protease (such as papain and trypsin), an organic acid a vitamin and a mineral (zinc).

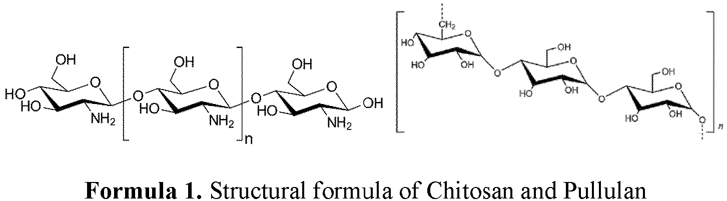

The proteolytic activity of papain and trypsin makes the stability of these formulations questionable, since these proteases are known to quickly hydrolyze the peptide bond of lysozyme, which therefore undergoes degradation (C R Hebd Seances Acad Sci. 1956 Dec 5;243(23):1926-8; doi.org/10.1016/0003-9861(56)90050-9). Moreover, the claimed C1-C4 polysaccharides interfere with the proteins present in the formulation, since chitosan is a basic compound and interacts with ovotransferrin, which has an isoelectric point of 6.0 and both polysaccharides react with the amino groups of lysozyme and ovotransferrin resulting in the formation of Schiff bases and of conjugates through a Maillard reaction (Food Hydrocolloids, Volume 71, October 2017, Pages 1-7, doi.org/10.1016/j.foodhyd.2017.04.026). These interactions among the selected excipients are responsible for the low stability of these formulations.

Several other formulations were further developed containing lysozyme and chitosan or carboxymethyl chitosan (Formula 2) as thickening agents (CN104001038; CN107715106).

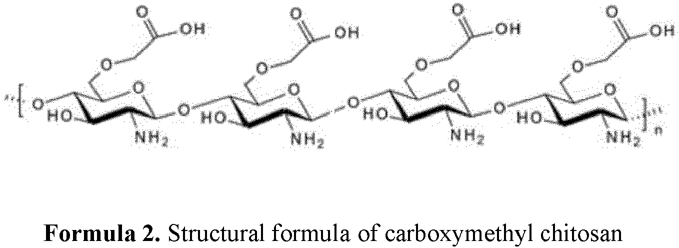

These complex formulations, which contain, in addition to lysozyme and carboxymethyl chitosan, several other natural components of vegetal origin, are not gels but solutions.

A new gel formulation containing human serum albumin in association with antimicrobial agents, including lysozyme, is disclosed in US2012164087; the claimed gel compositions contain 30 to 95% ethanol by weight. The role of ethanol in the preparation of a gel formulation with lysozyme C type is disclosed in US9757328: in fact, lysozyme itself is a gelling substance when treated with an alcohol/water mixture in a range from 80/20 to 60/40 and, therefore, it can be formulated without the addition of other gelling substances. However, these formulations have severe drawbacks since the use of such a high concentration of ethanol is not suitable for the dermatologic use (J Occup Med Toxicol 3, 26 (2008), doi: 10.1186/1745-6673-3-26). Moreover, the present Applicant experimentally found that the rheological characteristic (*i.e.,* viscosity) of these ethanolic formulations are not stable over time, even when stored at room temperature.

To the best of our knowledge, a stable gel formulation containing active ingredients lysozyme and ovotransferrin stable at physiological skin pH values of 4.1-5.8 using a non-polysaccharide thickening agent without the use of a surfactant (anionic or cationic) was never disclosed.

It should be noted that the use of a ionic surfactant utilized in combination with a thickening agent for the preparation of aqueous gel formulation (WO2005048962) should be avoided since it interferes with the secondary and tertiary structure of lysozyme and ovotransferrin, therefore resulting in the unfolding and denaturation of these proteins.

The Applicant carried out extensive studies with the aim to solve the above-mentioned problems and developed an antimicrobial gel active against acne caused by Gram-positive and Gram-negative microorganisms. The gel formulations of the invention employ lysozyme and ovotransferrin as active ingredients, are suitable for dermatological use and stable at room temperature over time, makes no use of ethanol as gelling agent, and has stable properties in term of viscosity and enzymatic/antimicrobial activity.

### Summary of the invention

The present invention discloses new stable anti-acne gel formulations comprising lysozyme, ovotransferrin, fatty ethoxylated dimeric urethanes , a preservative, water and optionally one or more humectants. The combination of Lysozyme and ovotransferrin provides synergistic antimicrobial effects against the microorganisms playing relevant role in the pathophysiology of *acne vulgaris.* Surprisingly, the gel formulations of the invention also maintain stable properties in term of viscosity and of antimicrobial action over time.

### Detailed description of the invention

Gels are jelly-like colloidal systems in which a liquid is dispersed in a solid medium. Since Lysozyme and ovotransferrin have different isoelectric points (pH=11 for lysozyme and pH=6 for ovotransferrin), it is critical to develop a stable gel formulation containing them. This means that the two proteins, at the normal skin pH value of 4.1-5.8, present a very different water solubility and therefore a high probability of precipitation in water-based gel formulations under storage conditions at room temperature. The tested formulations of the prior art, containing ethanol, pullulan, chitosan and carboxymethyl chitosan as gelling agent, showed low stability and unstable rheologic characteristics.

It has now surprisingly been found that both proteins can be formulated together in a stable and homogeneous gel using as a water-soluble non-ionic thickener a fatty ethoxylated dimeric urethane, providing clear gel formulations, in which the activities of lysozyme and ovotransferrin are stable over time. Furthermore, at the employed concentrations, said formulations are active against *Cutibacterium acnes, Corynebacterium minutissimum, Corynebacterium diphtheriae* and *Staphylococcus aureus.* Moreover, the antimicrobial activity of lysozyme is enhanced by the simultaneous presence of ovotransferrin, thus confirming an antimicrobial synergy therebetween.

The Applicant developed several formulations and found that the best stability in terms of enzymatic activity (determined using the *Micrococcus luteus* tests) and viscosity are obtained by using the active ingredients in the following weight percentages:
- fatty ethoxylated dimeric urethane from 2 to 8%, preferably 4%,
- lysozyme HCl from 1.5 to 3%, preferably 2.3%;
- ovotransferrin from 0.007 to 15.8%, preferably 0.58%.

According to the invention, the fatty ethoxylated dimeric urethanes include the compounds identified by the registry numbers 851820-35-4 (known with the trade name of Dermothix-100) and 53533-75-8 (known with the trade name of Dermothix-75), Dermothix-100 is preferably used.

Preferred formulations may also contain also a humectant selected from propylene glycol and glycerol in a percentage ranging from 5 to15% by weight, preferably 10%. These aqueous gel formulations contain water in an amount ranging from 70 to 80% by weight, preferably 73% and a preservative in a percentage ranging from 0.1 to 0.2% by weight, preferably 0.1%. The preservatives which can be used in these gel formulations may include, but are not limited to, sodium benzoate, glycol ethers, such as phenoxyethanol and caprylyl glycol and organic acids, such as benzoic acid, sorbic acid, levulinic acid and anisic acid.

The formulations of the invention may be used for the preparation of acne cleansing wet wipes and medicated pimple patches. The following experimental section and examples provide evidence of the utility of the formulations as antiseptic and antibacterial agents effective in decreasing the number of bacteria on the surface of the skin and in the treatment and prevention of *acne vulgaris.*

### Experimental section

- **Materials and Methods** Dermothix-100 and 75 were supplied by Alzo International Inc.
- Sodium benzoate was supplied by Sigma (Code number 18106)
- Lysozyme HCl was supplied by Bioseutica BV, The Netherland
- Ovotransferrin was supplied by Bioseutica BV, The Netherland
- Propylene glycol was supplied by Sigma (Code number 8.22324)
- Glycerol was supplied by Honeywell (Code number G7757)
- Tryptone agar plates (10 g/L tryptone, 15 g/L agar, sterilized by autoclaving)
- Sterile Petri dishes (92x16 mm, supplied by Sarstedt, Code number 82.1473.001)
- Sterile brain heart infusion (BHI) broth
- Sterile peptone water
- Sterile modified reinforced clostridial (RCM) broth
- Sterile cell spreaders (supplied by Sarstedt, Code number 86.1569.005)
- GasPak EZ anaerobic pouch system (supplied by VWR, Code number CA90003-648)
- GasPak anaerobic sachets (supplied by VWR, Code number CA 90003-642)
- *Propionibacterium acnes* (renamed as *Cutibacterium acnes,* ATCC 11827)
- *Corynebacterium minutissimum* (ATCC 23348)
- *Corynebacterium diphtheria* (ATCC 13812)
- *Staphylococcus aureus* (ATCC 6538)

### Example 1

Dermothix-100 (800g) and sodium benzoate (20 g) were dissolved in water (10.0 Kg) under stirring at 70°C for 60'. After this time, a clear solution (portion A) was obtained, then the solution was cooled down at 25-30°C. A solution of lysozyme HCl (460 g), ovotransferrin (116 g), glycerol (2,0 Kg) and water (4,50 Kg) (portion B) was prepared in a separate vessel under stirring at room temperature. The "portion B" solution was added slowly (5-10° min) into the vessel containing the "portion A" solution, afterwards propylene glycol (2,0 Kg) was added. The obtained mixture was then diluted with water to the final weight of 20Kg. The mixture was maintained under stirring at room temperature to obtain a homogeneous mixture (30'), stirring was arrested to purge the obtained mixture from air bubbles.

The weight percentage composition of the obtained gel formulation is reported below in Table 1.

**Table 1. Gel composition**

| **Compound** | **Weight %** |
|---|---|
| Dermothix-100 | 4 |
| Water | Q.s. to 100 |
| Sodium benzoate | 0.1 |
| Lysozyme HCl | 2.3 |
| Ovotransferrin | 0.58 |
| Propylene glycol | 10 |
| Glycerol | 10 |

The above gel formulation presents at time zero a viscosity of 2,500-3,600 mPa·s (cps) at about 25°C.

The collection of technical stability data concerning this formulation was carried out at 25°C for 24 months, at 40°C for 24 months and at 54°C for 21 days. During these timeframes, this formulation was analyzed for the viscosity and for the enzymatic activity of lysozyme. At room temperature, this gel showed unchanged enzymatic activity and viscosity. The same gel stored at 40 and 54°C was checked only for the enzymatic activity and proved to be unchanged.

**Table 2. Technical stability data**

| **Storage temperature** | **Time (days)** | **Enzymatic activity (*Micrococcus luteus* test; FIP unit/mg of product)** | **Viscosity** (mPa·s) |
|---|---|---|---|
| 25°C | 1 | 1.013 | 2.548 |
| | 2 | 873 | 2.788 |
| | 5 | 1.013 | 2.810 |
| | 15 | - | 3.300 |
| | 300 | 1.061 | 3.545 |
| | 742 | 1.064 | 3.090 |
| 40°C | 0 | 1.070 | - |
| | 8 | 1.087 | - |
| | 330 | 1.163 | - |
| | 742 | 1.125 | - |
| 54°C | 0 | 1.013 | - |
| | 5 | 1.013 | - |
| | 12 | 1.074 | 5.421 |
| | 21 | 1.099 | 5.100 |

### Microbiological tests for the efficacy of the gel formulation of the invention against Cutibacterium acnes, Corynebacterium minutissimum, Corynebacterium diphtheriae and Staphylococcus aureus

In this section, the gel containing lysozyme, ovotransferrin, sodium benzoate, propylene glycol, Dermothix-100 and glycerin, was microbiologically tested against microorganisms which play an important role in the pathophysiology of the common skin disease *acne vulgaris: Cutibacterium acnes, Corynebacterium minutissimum, Corynebacterium diphtheriae* and *Staphylococcus aureus.*

The negative control in the experiments was provided by formulations comprising some excipients but without lysozyme, ovotransferrin and/or sodium benzoate. In the preliminary experiments, different growth media were tested for the purpose of supporting the growth of the tested microorganisms, at the same time causing the minimum interference with lysozyme. To this purpose, a growth medium with the addition of tryptone agar provided the best solution. An application rate of 1g/plate of gel was considered to be appropriate. The experimental data were based on the use of tryptone agar as the growth medium at a gel application rate of 1 g/plate.

### Tested gel samples

Sample 1: gel formulation of the invention
Sample 2: sodium benzoate and glycerin
Sample 3: glycerin only, placebo
Sample 4: Benzoyl^{®}5; a commercial acne gel from Columbia Laboratories Canada Inc., containing 5% benzoyl peroxide (lot 1J01, Exp. 2024 SE)
Sample 5: sterile lysozyme and OT solution with the same concentrations as Sample 1 was prepared and included in each of the trials. The treatment with the application of this solution was designated as "Lyso/OT"

### General Procedures

- The inoculum of the following cultures was prepared:
   ∘ *Cutibacterium acnes* (ATCC 11827) was inoculated in RCM broth and incubated at 37°C anaerobically for three days.
   ∘ Staphylococcus aureus (ATCC 6538) was incubated into BHI broth at 35°C overnight aerobically.
   ∘ *Corynebacterium minutissimum* (ATCC 23348) and *Corynebacterium diphtheria* (ATCC 13812) were inoculated in BHI broth and incubated at 35°C aerobically for three days.
- On the day of the experiments, the cultures were diluted to levels at which 0.1 ml of the culture would result in 30-300 colonies. In some cases, 2-3 levels of the diluted cultures were tested to ensure that at least one of the dilutions fell within the valid colony count range.
- 0.1 ml of the above diluted cultures was spread onto the tryptone agar plates.
- Weighed 1±0.05 g of the gels or pipetted 1 ml of the Lyso/OT solution were added and spread across the plates.
   ∘ Negative controls for all four types of gels were included in each experiment to determine any growth in the gels.
   ∘ All the inoculated samples and negative controls were prepared in triplicates.
- Plates were incubated under the same conditions as described for the preparation of the inoculum.
- The colonies on the plates were counted after incubation.

The results of these microbiological tests are reported below.

### Cutibacterium acnes

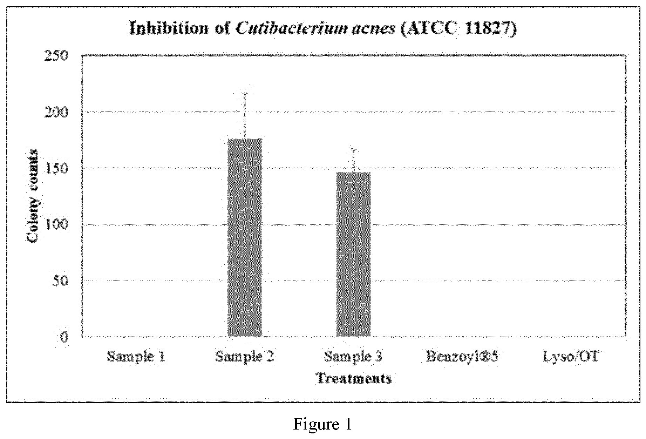

*Cutibacterium acnes* is one of the strains that cause acnes. At the inoculation level used, the colony counts for the plates with the application of Samples 2 and 3 were 176 and 146, respectively (Figure 1). With the addition of Samples 1, 4 and 5, the colony counts were reduced to <1 CFU/plate.

Sample 1: complete formulation with lysozyme, OT, sodium benzoate and glycerin; Sample 2: sodium benzoate and glycerin; Sample 3: glycerin only, placebo; Sample 4: Benzoyl^{®}5 gel: a commercial product from Columbia Laboratories Canada Inc., containing 5% benzoyl peroxide; Sample 5: a sterile lysozyme and OT solution with the same concentrations as in Sample 1.

### Corynebacterium minutissimum

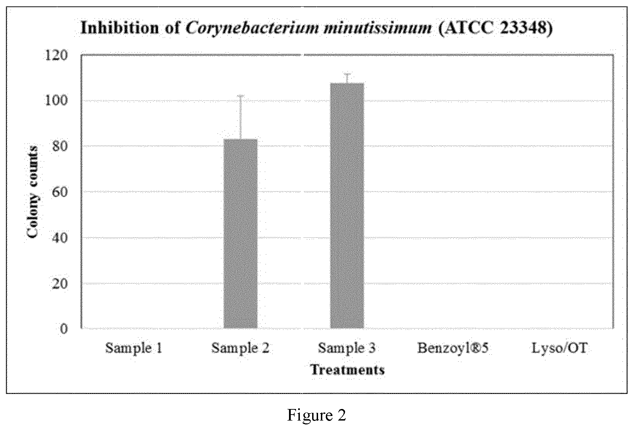

*Corynebacterium minutissimum* is associated with skin rash, known as erythrasma. The colony counts were 107 for the plates treated with Sample 3 and 83 with Sample 2 (Figure 2). When Samples 1, 4 and 5 were applied, no growth was observed on any of the agar plates (<1 CFU).

Sample 1: complete formulation with lysozyme, OT, sodium benzoate and glycerin; Sample 2: sodium benzoate and glycerin; Sample 3: glycerin only, placebo; Sample 4: Benzoyl^{®}5 gel: a commercial product from Columbia Laboratories Canada Inc., containing 5% benzoyl peroxide; Sample 5: a sterile lysozyme and OT solution with the same concentrations as in Sample 1.

### Corynebacterium diphtheriae

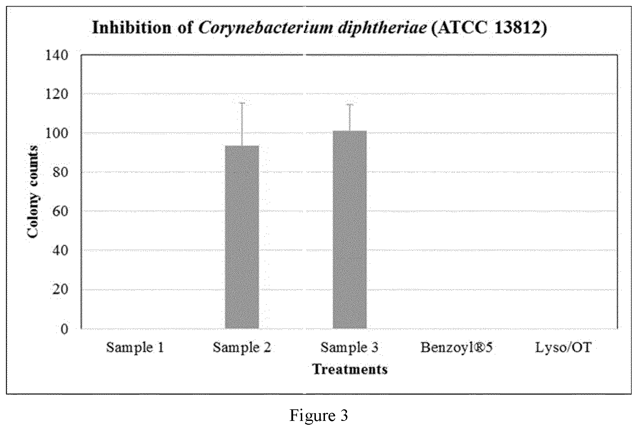

*Corynebacterium diphtheriae* is another member of the *Corynebacterium* genera that causes infections in the mucus membranes and skins. In this study, the growth of the inoculated cells was inhibited by Samples 1, 4 and 5 (Figure 3). The colony counts for plates treated with Samples 2 and 3 were 93 and 101, respectively.

Sample 1: complete formulation with lysozyme, OT, sodium benzoate and glycerin; Sample 2: sodium benzoate and glycerin; Sample 3: glycerin only, placebo; Sample 4: Benzoyl^{®}5 gel: a commercial product from Columbia Laboratories Canada Inc., containing 5% benzoyl peroxide; Sample 5: a sterile lysozyme and OT solution with the same concentrations as in Sample 1.

### Staphylococcus aureus

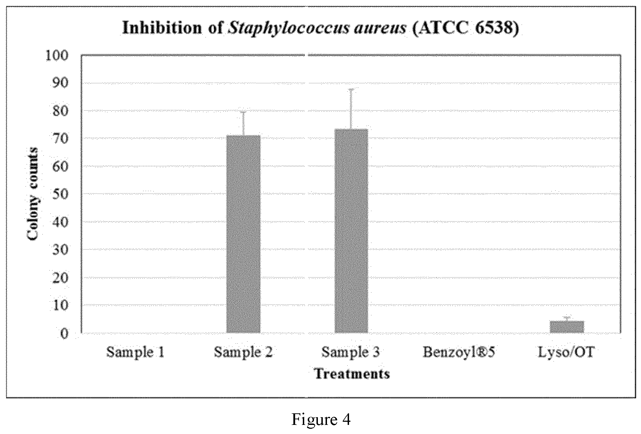

*Staphylococcus aureus* can cause skin and soft tissue infections in humans. In this study, 71 and 73 colonies were observed on the plates with the application of Samples 2 and 3 (Figure 4). With the addition of Samples 1 and 4, no growth was observed. Four colonies were recovered in the plate treated with the Sample 5.

Sample 1: complete formulation with lysozyme, OT, sodium benzoate and glycerin; Sample 2: sodium benzoate and glycerin; Sample 3: glycerin only, placebo; Sample 4: Benzoyl^{®}5 gel: a commercial product from Columbia Laboratories Canada Inc., containing 5% benzoyl peroxide; Sample 5: a sterile lysozyme and OT solution with the same concentrations as in Sample 1.

Under the experimental conditions, the formulation of the invention (Sample 1) was found to be effective against all four strains of the pathogens tested.

## Claims

1. Gel formulations for topical use containing lysozyme, ovotransferrin, fatty ethoxylated dimeric urethanes, a preservative, water and optionally one or more humectants.

2. Gel formulation according to claim 1 wherein lysozyme, as lysozyme base or hydrochloride, is present in a range from 1.5 to 3% by weight.

3. Gel formulations according to claim 1 wherein ovotransferrin is present in a range from 0.007 to 15.8% by weight.

4. Gel formulations according to claim 1 wherein the fatty ethoxylated dimeric urethanes where the alcohol is a 100 mole ethoxylated stearyl alcohol or a 75 mole ethoxylated stearyl alcohol or a mixture thereof, and are present in a range from 2 to 8% by weight.

5. Gel formulations according to claim 1 wherein the employed preservatives are selected from sodium benzoate, phenoxyethanol, caprylyl glycol, benzoic acid, sorbic acid, levulinic acid and anisic acid.

6. Gel formulations according to claim 1 wherein water is present in a range from 70 to 80% by weight.

7. Gel formulations according to claim 1 wherein the humectants are selected from glycerol and propylene glycol, each present in a range from 5 to 15% by weight.

8. Gel formulations according to claim 1 having at time zero a viscosity of 2,5-3,6 Pa.s at 25°C.

9. Cleansing wet wipes and medicated pimple patches comprising the formulations of claims 1-8.

10. The gel formulations of claims 1-8 for use in the topical treatment and prevention of acne vulgaris and for reducing the number of bacteria on the surface of the skin.

## Patentansprüche

1. Gelformulierungen zur topischen Anwendung, enthaltend Lysozym, Ovotransferrin, Fettalkohol-ethoxylierte dimere Urethane, ein Konservierungsmittel, Wasser und gegebenenfalls ein oder mehrere Feuchthaltemittel.

2. Gelformulierung nach Anspruch 1, wobei Lysozym als Lysozymbase oder Hydrochlorid in einem Bereich von 1,5 bis 3 Gew.-% vorliegen.

3. Gelformulierungen nach Anspruch 1, wobei Ovotransferrin in einem Bereich von 0,007 bis 15,8 Gew.-% vorliegen.

4. Gelformulierungen nach Anspruch 1, wobei die Fettalkohol-ethoxylierten dimeren Urethane, bei denen der Alkohol ein 100-Mol-ethoxylierter Stearylalkohol oder ein 75-Mol-ethoxylierter Stearylalkohol oder eine Mischung davon ist, in einem Bereich von 2 bis 8 Gew.-% vorliegen.

5. Gelformulierungen nach Anspruch 1, wobei die verwendeten Konservierungsmittel aus Natriumbenzoat, Phenoxyethanol, Caprylylglykol, Benzoesäure, Sorbinsäure, Levulinsäure und Anisinsäure ausgewählt sind.

6. Gelformulierungen nach Anspruch 1, wobei Wasser in einem Bereich von 70 bis 80 Gew.-% vorliegt.

7. Gelformulierungen nach Anspruch 1, wobei die Feuchthaltemittel ausgewählt sind aus Glycerin und Propylenglykol, die jeweils in einem Bereich von 5 bis 15 Gew.-% vorliegen.

8. Gelformulierungen nach Anspruch 1, die zum Zeitpunkt Null eine Viskosität von 2,5 bis 3,0 Pa.s bei 25°C aufweisen.

9. Reinigungstücher und medizinische Pickelpflaster, umfassend die Formulierungen nach den Ansprüchen 1 bis 8.

10. Gelformulierungen nach den Ansprüchen 1 bis 8 zur Verwendung bei der topischen Behandlung und Vorbeugung von Akne vulgaris und zur Verringerung der Anzahl von Bakterien auf der Hautoberfläche.

## Revendications

1. Formulations de gel à usage topique contenant du lysozyme, de l'ovotransferrine, des uréthanes dimériques éthoxylés gras, un conservateur, de l'eau et, éventuellement, un ou plusieurs humectants.

2. Formulation de gel selon la revendication 1, dans laquelle le lysozyme, sous forme de lysozyme base ou de chlorhydrate de lysozyme, est présent dans une plage de 1,5 à 3 % en poids.

3. Formulations de gel selon la revendication 1, dans lesquelles l'ovotransferrine est présente dans une plage de 0,007 à 15,8 % en poids.

4. Formulations de gel selon la revendication 1, dans lesquelles les uréthanes dimériques éthoxylés gras où l'alcool est un alcool stéarylique éthoxylé à 100 moles ou un alcool stéarylique éthoxylé à 75 moles ou un mélange de ceux-ci, et sont présents dans une plage de 2 à 8 % en poids.

5. Formulations de gel selon la revendication 1, dans lesquelles les conservateurs utilisés sont choisis parmi le benzoate de sodium, le phénoxyéthanol, le caprylyl glycol, l'acide benzoïque, l'acide sorbique, l'acide lévulinique et l'acide anisique.

6. Formulations de gel selon la revendication 1, dans lesquelles l'eau est présente dans une plage de 70 à 80 % en poids.

7. Formulations de gel selon la revendication 1, dans lesquelles les humectants sont choisis parmi le glycérol et le propylène glycol, chacun présent dans une plage de 5 à 15 % en poids.

8. Formulations de gel selon la revendication 1, ayant au temps zéro une viscosité de 2,5 à 3,6 Pa.s à 25 °C.

9. Lingettes nettoyantes humides et timbres médicinaux contre les boutons, comprenant les formulations des revendications 1 à 8.

10. Formulations de gel des revendications 1 à 8, pour une utilisation dans le traitement et la prévention topiques de l'acné vulgaire et pour réduire le nombre de bactéries à la surface de la peau.
